(19)

Europäisches
Patentamt

European
Patent Office

Office européen
des brevets

(11) **EP 4 593 765 B1**

(12) **EUROPEAN PATENT SPECIFICATION**

(45) Date of publication and mention
of the grant of the patent:
**13.05.2026   Bulletin 2026/20**

(21) Application number: **23793472.4**

(22) Date of filing: **02.10.2023**

(51) International Patent Classification (IPC):
*A61F 2/30* (2006.01)      *A61F 2/34* (2006.01)
*A61F 2/36* (2006.01)      *A61L 27/54* (2006.01)

(52) Cooperative Patent Classification (CPC):
**A61F 2/3094; A61F 2/34; A61F 2/36; A61L 27/54;**
A61F 2310/0052; A61L 2300/104; A61L 2300/404;
A61L 2400/12

(86) International application number:
**PCT/IB2023/059847**

(87) International publication number:
**WO 2024/069605 (04.04.2024 Gazette 2024/14)**

(54) **METHOD OF MANUFACTURING A SILVER DENDRITE PLATFORM FOR BIOMEDICAL APPLICATIONS**

VERFAHREN ZUR HERSTELLUNG EINER SILBERDENDRITPLATTFORM FÜR BIOMEDIZINISCHE ANWENDUNGEN

PROCÉDÉ DE FABRICATION D'E PLATEFORME DE DENDRITES D'ARGENT POUR APPLICATIONS BIOMÉDICALES

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority:  **30.09.2022  IT 202200020145**

(43) Date of publication of application:
**06.08.2025   Bulletin 2025/32**

(73) Proprietor: **Consiglio Nazionale Delle Ricerche
00185 Roma (IT)**

(72) Inventors:
• **CONOCI, Sabrina
  00185 Roma (IT)**
• **IRRERA, Alessia
  00185 Roma (IT)**
• **CALABRESE, Giovanna
  00185 Roma (IT)**
• **SCIUTO, Emanuele Luigi
  00185 Roma (IT)**
• **LEONARDI, Antonio Alessio
  00185 Roma (IT)**
• **SPINELLA, Corrado Rosario
  00185 Roma (IT)**
• **FRANCO, Domenico
  98070 Capri Leone (IT)**
• **GUGLIELMINO, Salvatore
  95127 Catania (IT)**
• **RIZZO, Maria Giovanna
  90017 Santa Flavia (IT)**

(74) Representative: **Maccalli, Marco et al
Maccalli & Pezzoli S.r.l.
Via Settembrini, 40
20124 Milano (IT)**

(56) References cited:
**WO-A1-2013/059745     WO-A1-2021/239877**

• **ABOUDA-LACHIHEB MANEL ET AL: "The dual
role of silver during silicon etching in HF
solution", NANOSCALE RESEARCH LETTERS
2012 7:455, 13 August 2012 (2012-08-13),
XP093035286, Retrieved from the Internet
<URL:https://link.springer.com/content/pdf/
10.1186/1556-276X-7-455.pdf?pdf=button>
[retrieved on 20230328]**

EP 4 593 765 B1

• ALHMOUD HASHIM ET AL: "Antibacterial properties of silver dendrite decorated silicon nanowires", RSC ADVANCES, vol. 6, no. 70, 5 July 2016 (2016-07-05), GB, pages 65976 - 65987, XP093035288, ISSN: 2046-2069, DOI: 10.1039/C6RA13734B

## Description

Technical Field

The present invention relates to a method of manufacturing a silver dendrite platform for biomedical applications. The invention further relates to a prosthetic device comprising said silver dendrite platform.

Background Art

One of the most dreaded complications following the implantation of a prosthetic device is periprosthetic infection, as the metal surface of the implant is an ideal breeding ground for bacteria and the formation of a biofilm.

In Italy the rate of prosthetic infection is 2-5% and the cost for a revision of an infected prosthesis is 4.8 times that of a primary implant. In addition, periprosthetic infection causes bone-destroying processes that lead not only to the loosening of the prosthesis but also to the progressive destruction of the joint as a whole.

Among the most widely used systems to reduce biofilm formation in orthopaedic implants is the use of metals, including silver, copper, gold, titanium and zinc, in the preparation of implantable devices. At the nanoparticle scale, however, the most interesting metals seem to be copper and, above all, silver. In fact, silver nanoparticles have been widely studied in recent years because of their ability to reduce the adhesion of fungi and bacteria.

However, although silver nanoparticles have shown exceptional antibacterial and anti-biofilm properties, they present cytotoxicity problems that severely limit their use, especially in the biomedical field. Researchers are still trying to determine the optimal amount and size of silver nanoparticles to be used in implantable materials or for doping implantable materials in order to avoid possible adverse effects. Unfortunately, this is one of the most important limitations for the use of silver nanostructures in the manufacture of implantable orthopaedic devices to date.

Manel Abouda-Lachiheb et al., "The dual role of silver during silicon etching in HF solution", Nanoscale Research Letters 2012 report that during silicon etching, silver was subjected to have a controversial role. Some researchers debate that silver protects silicon, and, at the same time, other ones confirm that silver catalyzes silicon underneath. The authors give experimental results arguing the dual role that silver has during the formation of silicon nanostructures, and they give a proof that the role of silver depends on the experimental details and the intrinsic properties of silver during its deposition on the silicon wafer.

The object of the invention is to overcome the problems and limitations of current prosthetic devices by providing a platform for biomedical applications in the orthopaedic field that exhibits exceptional antimicrobial capabilities while having good compatibility with eukaryotic cells.

This and other objects are achieved with the platform for biomedical applications obtained by the method as claimed in the appended claims.

Summary of Invention

The method of manufacturing a silver dendrite platform according to the invention is defined in claim 1 and comprises the steps of:

- providing a silicon wafer stripped of its native surface silicon oxide layer,
- immersing said silicon wafer in a solution of silver nitrate and hydrofluoric acid.

In said step of immersing the silicon wafer in the solution of silver nitrate and hydrofluoric acid, the silver nitrate dissociates into $NO_3^-$ anions and $Ag^+$ cations, and said $Ag^+$ cations then precipitate on the silicon wafer, thus resulting in the formation of multiple silver dendrites. Preferably, said step of immersing the silicon wafer in the solution of silver nitrate and hydrofluoric acid is carried out at room temperature and for essentially 30 minutes.

The method further comprises the step of separating the silver dendrites from the silicon wafer thus obtaining a layer of isolated silver dendrites, i.e., the silver dendrite platform.

According to the invention, in the step of immersing the silicon wafer in the solution of silver nitrate and hydrofluoric acid, a matrix of silicon nanowires forms on the surface of the silicon wafer by the process of metal-assisted chemical etching, wherein the silver formed on the surface of the wafer acts as catalyst for said chemical etching.

Advantageously, the silver dendrites of the platform obtained by the method according to the invention have a fractal-like structure.

The method makes it possible to obtain silver dendrites having a length between 50 nm and 20 $\mu$m and a thickness between 10 $\mu$m and 30 $\mu$m.

Advantageously, the silver dendrite platform obtained by the method according to the invention exhibits excellent antimicrobial capabilities and has shown good compatibility with eukaryotic cells, therefore being particularly suitable for biomedical applications, especially for prosthetic devices such as orthopaedic or dental implants.

The pronounced antimicrobial activity of the silver dendrites of the platform is also due to their enormous surface-to-volume ratio, due to their fractal-like structure, which allows the surface area available for antibacterial activity to be greatly increased for the same volume. Furthermore, it can be hypothesised that the absence of cytotoxicity in silver dendrites may be due to the absence or low presence of silver ions in the biological environment with which they are in contact (cell matrix, cells...).

Brief Description of Drawings

These and other features and advantages of the present invention will become evident from the following description of preferred embodiments given by way of nonlimiting examples with reference to the annexed drawings, in which parts identified with identical or similar reference numerals denote parts having identical or similar function and construction, and in which:

| | |
|---|---|
| Figs.1a-1b | schematically represent an intermediate step and the final step of the method of manufacturing the platform according to an embodiment of the invention; |
| Fig.2 | shows an SEM cross-sectional image of silver dendrites, having an average thickness of $15 \pm 5$ $\mu$m, on a matrix of silicon nanowires (Si NW) of about 2 $\mu$m in height, obtained by the method of manufacturing the platform according to an embodiment of the invention; |
| Fig.3 | shows an in-plane SEM image of silver dendrites obtained by the method of manufacturing the platform according to an embodiment of the invention; |
| Fig.4 | shows a prosthetic device (femoral prosthesis) comprising platforms of silver fractals according to the invention; |
| Figs.5a-5b | show photos of the results of the antibacterial activity on bacterial cultures of *S. aureus* ATCC 29213 and *P. aeruginosa* ATCC 27853, in the absence (Figure 5a) and presence (Figure 5b) of silver dendrite platforms; |
| Figs.6a-6b | show the graphs of vital count results, in CFU/ml (colony-forming units per millimetre), performed on bacterial cultures of *S. aureus* ATCC 29213 and *P. aeruginosa* ATCC 27853 in the absence and presence of silver dendrite platforms (Figure 6a) and the bacterial viability compared to the cultures in the absence of the bacterial platforms (bacterial viability vs CTR) (Figure 6b); |
| Fig.7 | shows the graph of the cell viability of eukaryotic cells (hFOB, human fetal osteoblasts) in the presence of silver dendrites as a percentage value compared to that in the absence of silver dendrites. |

Description of Embodiments

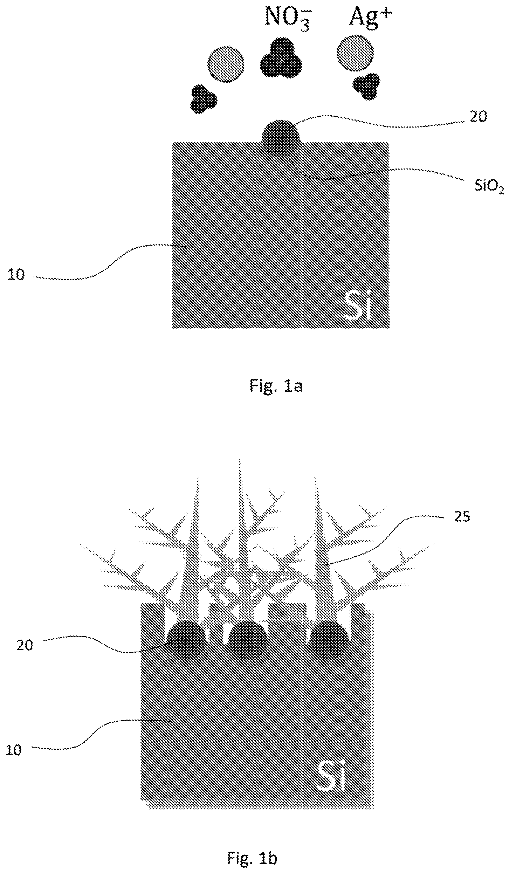

A method of manufacturing a silver dendrite platform for biomedical applications according to a preferred embodiment is described here below with reference to Figures 1a-b.

The platform is obtained on the surface of a silicon wafer 10. In particular, after an initial step of typical cleaning of the silicon wafer 10, for example by means of bathing in acetone for 5 minutes, in isopropanol for 5 minutes and rinsing with water, the silicon wafer 10 is preferably cleaned of organic contaminants by means of a UV-ozone treatment for 2 minutes. After cleaning, the native oxide covering the silicon wafer 10 is lifted off by using an aqueous solution of hydrofluoric acid 5%.

After said step, the wafer 10 is immersed in a solution containing 0.02 M silver nitrate ($AgNO_3$), as silver precursor, and 5 M hydrofluoric acid diluted in deionised water for 30 minutes at room temperature. In such solution, the silver salts dissociate into single $NO_3^-$ anions and $Ag^+$ cations, as shown in Figure 1a, and said $Ag^+$ cations precipitate as clusters on the silicon wafer. Thus, seeds of silver particles 20 form on the surface of the wafer, on which particles further $Ag^+$ ions accumulate, thereby forming, as shown in Figure 1b, silver dendrites 25, i.e., crystalline silver aggregates with an arborescent, fractal-like appearance.

During the same process, the silver particles also act as catalyst for the chemical etching of the underlying silicon, according to the process known as Metal-assisted Chemical Etching. This chemical etching causes, as a side product, the formation of a matrix of silicon nanowires (Si NW) 12 on the surface of the wafer 10.

The above-mentioned steps of formation of silver dendrites 25 and chemical etching are carried out at room temperature.

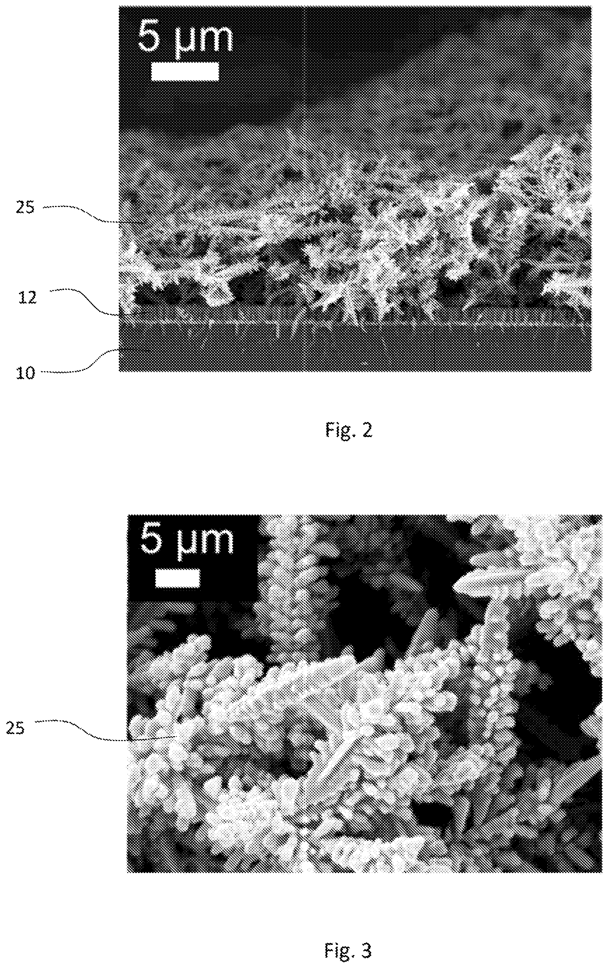

The silver dendrites 25 obtained by the method mentioned above have an average thickness of $15 \pm 5$ $\mu$m and the silicon nanowires 12 on which the dendrites develop have a length of approximately 2 $\mu$m, as shown in the SEM images in Figures 2 and 3. The same images also show that the obtained silver dendrites 25 are organised in a dense forest.

According to other embodiments of the method of manufacturing the platform, by changing the duration of the chemical etching from 3 seconds to 30 minutes it is possible to obtain silver dendrites with different structural and morphological characteristics. For example, it is possible to obtain dendrites having a length between 50 nm e 20 $\mu$m and a thickness between 10 and 30 $\mu$m.

The silver dendrites 25 are then separated from the silicon wafer 10, thereby obtaining a layer of isolated silver dendrites, i.e., a silver dendrite platform 30. The platform 30 obtained according to the method described above can be used in multiple biomedical applications, particularly in prosthetic devices such as orthopaedic or dental implants.

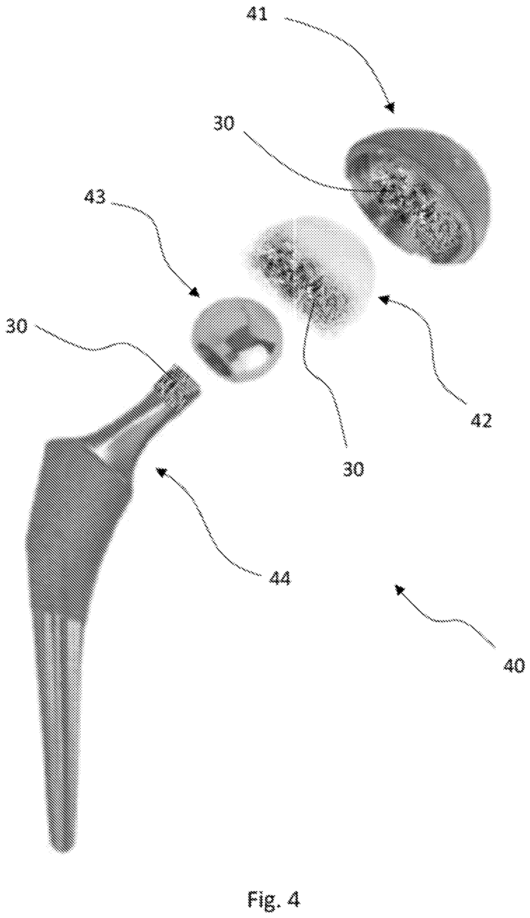

Referring to Figure 4, an example of a prosthetic device comprising the silver dendrite platform is a femoral prosthesis 40. Said prosthesis 40 comprises an acetabular cup 41, usually made of titanium, an acetabular insert 42, usually made of plastics or ceramics, a femoral head 43, usually made of metal or ceramics, and a femoral stem 44, usually made of titanium. The silver dendrite platform 30 is applied between the acetabular cup 41 and the acetabular insert 42, between the acetabular insert 42 and the femoral head 43 and between the femoral head 43 and the femoral stem 44.

The silver dendrite platform exhibits excellent antimicrobial capabilities and good compatibility with eukariotic cells, as evidenced by the results of microbiological and cellular analyses, some of which are reported below.

Microbiological and Cellular Results

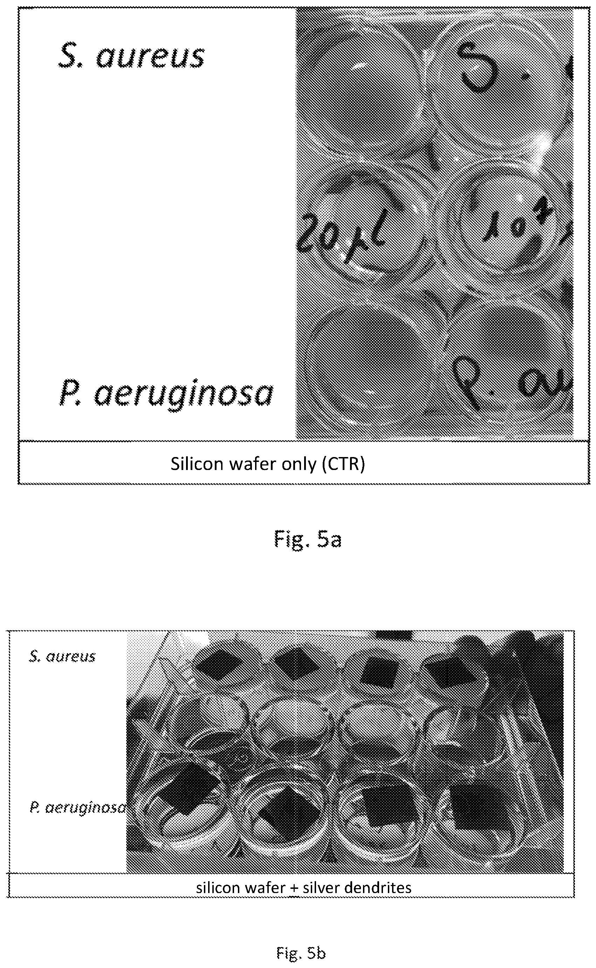

The results of the antibacterial activity on two different bacterial cultures, *S. aureus* ATCC 29213 (gram-positive) and *P. aeruginosa* ATCC 27853 (gram-negative), in the absence (Figure 5a) and presence (Figure 5b) of silver dendrite platforms, show that after 24 h incubation, only the *P. aeruginosa* culture, in the presence of silver dendrites, shows no bacterial growth, as indicated by the absence of turbidity in the culture medium. Conversely, the culture of *S. aureus* in the presence of silver dendrites shows bacterial growth, as indicated by the turbidity of the culture medium.

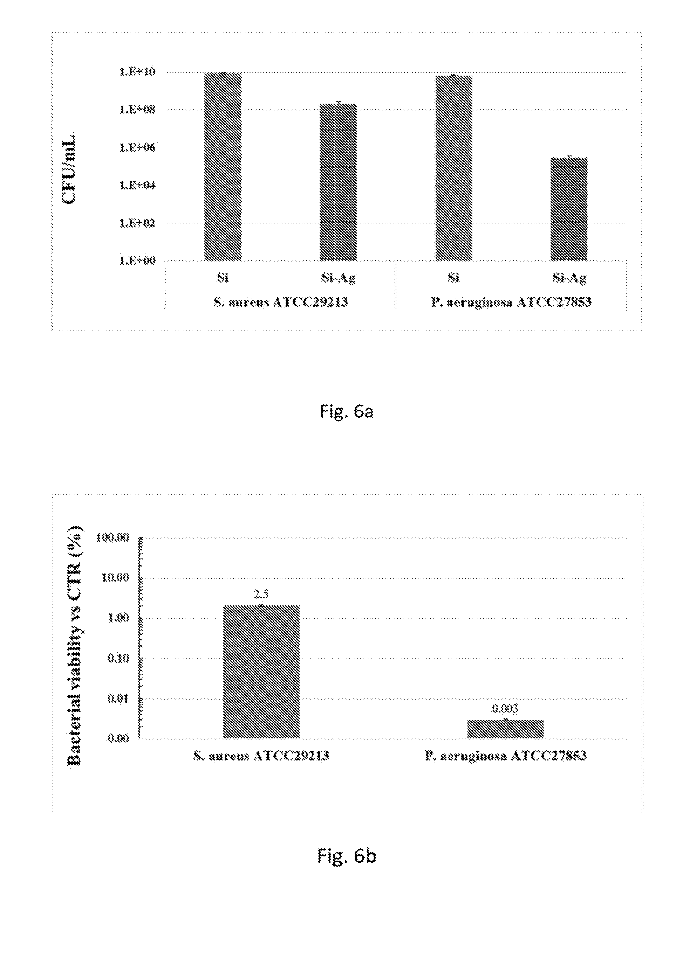

Furthermore, the viable count (CFU/mL) performed on the two different bacterial strains mentioned above indicated that *S. aureus* has a residual viability, in the presence of the silver dendrites, of 2.5% (a 97.5% reduction compared to the result without silver dendrites), whereas *P. aeruginosa* has a residual viability of 0.003% (a 99.997% reduction), as shown in the graphs in Figures 6a-b. The results therefore suggest a more effective antimicrobial activity of silver dendrites against the gram-negative bacterial strain.

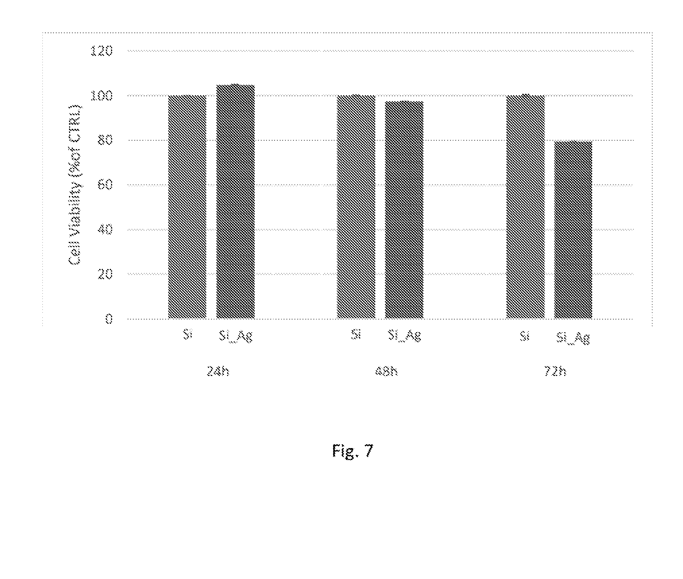

In addition, the data obtained from the assessment of the biocompatibility of eukariotic cells (hFOB, human fetal osteoblasts) in the presence of silver dendrites indicate the absence of cytotoxicity over time from 0 up to 72 hours. As shown in the graph in Figure 7, in fact, the viability measured in the presence of silver dendrites is above the 70% threshold, which discriminates biocompatibility from potential cytotoxicity (according to the ISO 10993 standard).

**Claims**

1. Method of manufacturing a silver dendrite platform (30), comprising the steps of:

    - providing a silicon wafer stripped of its native surface silicon oxide layer,
    - immersing said silicon wafer (10) in a solution of silver nitrate and hydrofluoric acid,
    wherein, in said step of immersing the silicon wafer in the solution of silver nitrate and hydrofluoric acid, the silver nitrate dissociates into $NO_3^-$ anions and Ag$^+$ cations, and said Ag$^+$ cations then precipitate on the silicon wafer, thus resulting in the formation of multiple silver dendrites (25),
**characterized in that** the silver dendrite platform is for biomedical applications,
and **in that** the method further comprises:

    - separating the silver dendrites from the silicon wafer thus obtaining the silver dendrite platform comprising a layer of isolated silver dendrites.

2. Method according to claim 1, wherein the obtained silver dendrites have a fractal-like structure.

3. Method according to claim 1 or 2, wherein in said step of immersing the silicon wafer in the solution of silver nitrate and hydrofluoric acid, a matrix of silicon nanowires (12) forms on the surface of the silicon wafer by the process of metal-assisted chemical etching, wherein the silver formed on the surface of the wafer acts as catalyst for said chemical etching.

4. Method according to any one of the preceding claims, wherein said step of immersing the silicon wafer in the solution of silver nitrate and hydrofluoric acid is carried out substantially at room temperature.

5. Method according to any one of the preceding claims, wherein said step of immersing the silicon wafer in the solution of silver nitrate and hydrofluoric acid is carried out for essentially 30 minutes.

6. Method according to any one of the preceding claims, wherein the obtained silver dendrites have a length between 50 nm and 20 μm and a thickness between 10 μm and 30 μm.

7. Method according to any one of claims 2 to 5, wherein the obtained silicon nanowires have a length of about 2 μm.

8. Prosthetic device (40) comprising at least one silver dendrite platform (30) obtained by the method according to any one of claims 1 to 7.

9. Prosthetic device according to claim 8, said device being a femoral prosthesis (40) comprising an acetabular cup (41), an acetabular insert (42), a femoral head (43) and a femoral stem (44), wherein the silver dendrite platform is applied between the acetabular cup and the acetabular insert and/or between the acetabular insert and the femoral head and/or between the femoral head and the femoral stem.

## Patentansprüche

1. Verfahren zum Herstellen einer Silberdendritplattform (30), umfassend die folgenden Schritte:

   - Bereitstellen eines Siliziumwafers, der von seiner nativen Oberflächen-Siliziumoxidschicht befreit ist,
   - Eintauchen des Siliziumwafers (10) in eine Lösung aus Silbernitrat und Flusssäure, wobei in dem Schritt des Eintauchens des Siliziumwafers in die Lösung aus Silbernitrat und Flusssäure das Silbernitrat in $NO_3^-$-Anionen und $Ag^+$-Kationen dissoziiert und sich die $Ag^+$-Kationen dann auf dem Siliziumwafer niederschlagen, was zur Bildung mehrerer Silberdentriten (25) führt,
   **dadurch gekennzeichnet, dass** die Silberdendritplattform für biomedizinische Anwendungen vorgesehen ist und dass das Verfahren ferner Folgendes umfasst:

   - Trennen der Silberdendriten von dem Siliziumwafer, wodurch die Silberdentritplattform erlangt wird, die eine Schicht aus isolierten Silberdendriten umfasst.

2. Verfahren nach Anspruch 1, wobei die erlangten Silberdendriten eine fraktalartige Struktur aufweisen.

3. Verfahren nach Anspruch 1 oder 2, wobei sich in dem Schritt des Eintauchens des Siliziumwafers in die Lösung aus Silbernitrat und Flusssäure eine Matrix aus Siliziumnanodrähten (12) auf der Oberfläche des Siliziumwafers durch den Vorgang des metallunterstützten chemischen Ätzens bildet, wobei das auf der Oberfläche des Wafers gebildete Silber als Katalysator für das chemische Ätzen wirkt.

4. Verfahren nach einem der vorhergehenden Ansprüche, wobei der Schritt des Eintauchens des Siliziumwafers in die Lösung aus Silbernitrat und Flusssäure im Wesentlichen bei Raumtemperatur durchgeführt wird.

5. Verfahren nach einem der vorhergehenden Ansprüche, wobei der Schritt des Eintauchens des Siliziumwafers in die Lösung aus Silbernitrat und Flusssäure wesentlich 30 Minuten lang durchgeführt wird.

6. Verfahren nach einem der vorhergehenden Ansprüche, wobei die erlangten Silberdendriten eine Länge zwischen 50 nm und 20 μm und eine Dicke zwischen 10 μm und 30 μm aufweisen.

7. Verfahren nach einem der Ansprüche 2 bis 5, wobei die erlangten Silizium-Nanodrähte eine Länge von etwa 2 μm aufweisen.

8. Prothesenvorrichtung (40), umfassend mindestens eine Silberdendritplattform (30), erlangt durch das Verfahren nach einem der Ansprüche 1 bis 7.

9. Prothesenvorrichtung nach Anspruch 8, wobei die Vorrichtung eine Femurprothese (40) ist, die eine Hüftgelenkpfannenschale (41), einen Hüftgelenkpfanneneinsatz (42), einen Femurkopf (43) und einen Femurschaft (44) umfasst,
   wobei die Silberdendritplattform zwischen der Hüftgelenkpfannenschale und dem Hüftgelenkpfanneneinsatz und/oder zwischen dem Hüftgelenkpfanneneinsatz und dem Femurkopf und/oder zwischen dem Femurkopf und dem Femurschaft aufgebracht ist.

## Revendications

1. Procédé de fabrication d'une plateforme de dendrites d'argent (30), comprenant les étapes de :

   - fourniture d'une tranche de silicium dépouillée de sa couche d'oxyde de silicium de surface native,
   - immersion de ladite tranche de silicium (10) dans une solution de nitrate d'argent et d'acide fluorhydrique,
   dans ladite étape d'immersion de la tranche de silicium dans la solution de nitrate d'argent et d'acide fluorhydrique, ledit nitrate d'argent se dissociant en anions $NO_3^-$ et en cations $Ag^+$, et lesdits cations $Ag^+$ se précipitant ensuite sur la tranche de silicium, ce qui entraîne la formation de multiples dendrites d'argent (25),
   **caractérisé en ce que** la plateforme de dendrites d'argent est destinée à des applications biomédicales, et **en ce que** le procédé comprend en outre :

   - la séparation des dendrites d'argent de la plaquette de silicium, ce qui permet l'obtention de la plateforme de dentrites d'argent comprenant une couche de dendrites d'argent isolées.

2. Procédé selon la revendication 1, lesdites dendrites d'argent obtenues présentant une structure de type fractale.

3. Procédé selon la revendication 1 ou 2, dans ladite étape d'immersion de la tranche de silicium dans la solution de nitrate d'argent et d'acide fluorhydrique, une matrice de nanofils de silicium (12) se formant à la surface de la tranche de silicium par le processus de gravure chimique assistée par métal, ledit argent formé à la surface de la tranche agissant comme un catalyseur pour ladite gravure chimique.

4. Procédé selon l'une quelconque des revendications précédentes, ladite étape d'immersion de la tranche de silicium dans la solution de nitrate d'argent et d'acide fluorhydrique étant effectuée sensiblement à température ambiante.

5. Procédé selon l'une quelconque des revendications précédentes, ladite étape d'immersion de la tranche de silicium dans la solution de nitrate d'argent et d'acide fluorhydrique étant effectuée pendant sensiblement 30 minutes.

6. Procédé selon l'une quelconque des revendications précédentes, lesdites dendrites d'argent obtenues présentant une longueur comprise entre 50 nm et 20 $\mu$m et une épaisseur comprise entre 10 $\mu$m et 30 $\mu$m.

7. Procédé selon l'une quelconque des revendications 2 à 5, lesdits nanofils de silicium obtenus présentant une longueur d'environ 2 $\mu$m.

8. Dispositif prothétique (40) comprenant au moins une plateforme de dendrites d'argent (30) obtenue par le procédé selon l'une quelconque des revendications 1 à 7.

9. Dispositif prothétique selon la revendication 8, ledit dispositif étant une prothèse fémorale (40) comprenant une cupule acétabulaire (41), un insert acétabulaire (42), une tête fémorale (43) et une tige fémorale (44),
ladite plateforme de dendrites d'argent étant appliquée entre la cupule acétabulaire et l'insert acétabulaire et/ou entre l'insert acétabulaire et la tête fémorale et/ou entre la tête fémorale et la tige fémorale.

Fig. 1a

Fig. 1b

Fig. 2

Fig. 3

Fig. 4

Fig. 5a

Fig. 5b

Fig. 6a

Fig. 6b

Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MANEL ABOUDA-LACHIHEB et al.** The dual role of silver during silicon etching in HF solution. *Nanoscale Research Letters*, 2012